# EUROPEAN PATENT SPECIFICATION

(11) **EP 3 962 741 B1**
(45) Date of publication and mention of the grant of the patent: **18.06.2025**
(21) Application number: 20799031.8
(22) Date of filing: 29.04.2020
(51) Int. Cl.: B32B 27/08, B32B 7/12, B32B 27/30, B32B 27/32, A61L 2/08, B32B 27/18, B32B 27/20, B32B 27/22, B32B 27/36, B65D 1/02

(54) **MULTI-LAYER ARTICLE FOR STORING A PRODUCT**
MEHRSCHICHTIGER GEGENSTAND ZUR LAGERUNG EINES PRODUKTS
ARTICLE MULTICOUCHE SERVANT À STOCKER UN PRODUIT

(30) Priority: 02.05.2019 US 201962842305 P; 27.04.2020 US 202016859647
(43) Date of publication of application: 09.03.2022
(73) Proprietor: Honeywell International Inc., Charlotte, NC 28202 (US)
(72) Inventor: PRATT, Jeff, Charlotte, North Carolina 28202 (US); SKWAREK, Gary M., Charlotte, North Carolina 28202 (US); WOODS, Joshua Allen, Charlotte, North Carolina 28202 (US)
(74) Representative: Stepney, Gregory John
(86) International application number: PCT/US2020/030371
(87) International publication number: WO 2020/223286

(56) References cited:
- WO-A1-93/03922
- WO-A2-2005/082722
- US-A1- 2003 031 891
- US-A1- 2005 077 202
- US-A1- 2005 186 376
- US-A1- 2006 014 022
- US-A1- 2008 023 064
- US-A1- 2015 158 644

## Description

### TECHNICAL FIELD

The present disclosure generally relates to a multi-layer article for storing a product. More specifically, this disclosure relates to a multi-layer article that includes a first layer comprising polychlorotrifluoroethylene and/or copolymer of polychlorotrifluoroethylene and vinylidene fluoride, a tie layer comprising an ethylene acrylate, and a second layer comprising polyester, polycarbonate, polypropylene, polyethylene, or combinations thereof.

### BACKGROUND

A wide variety of thermoplastic polymers and containers formed from such thermoplastic polymers are known. Important physical characteristics of such containers include barrier properties, including barriers to gas, aroma, and/or vapor such as water vapor, as well as physical characteristics, such as toughness, clarity, wear and weathering resistances, light-transmittance and chemical inertness. These properties are especially important in packaging applications for food or medical products. For example, WO9303922A1 describes a multilayer film structure.

Many fluoropolymer materials are commonly known for their excellent moisture and vapor barrier properties, as well as their chemical resistance, and therefore are desirable components of packaging articles, particularly lidding films, blister packages and bottle containers. In addition, fluoropolymers exhibit high thermal stability and excellent toughness. However, such use of fluoropolymers is restricted to specialty packaging applications due to their relatively high cost. A suitable means of reducing the cost of a packaging material fabricated from a costly polymer is to form multilayer structures in which the polymer is laminated with other, less costly polymer layers. This approach is particularly desirable for the fluoropolymer packaging applications since a thin layer of the fluoropolymer is often all that is needed to take advantage of the desirable properties of the fluoropolymer while minimizing the cost. However, fluoropolymers do not adhere strongly to most other polymers. In fact, most fluoropolymers are known for their non-stick characteristics. This is very disadvantageous, because poor bond strength between layers can result in the delamination of multilayer structures.

There is also a continuing need in the art for further improvements in molded articles formed from fluoropolymers. Particularly desirable are lightweight, non-breakable structures with good clarity, a high moisture barrier, good chemical resistance and low water vapor and gas transmission. More particularly, there is a need in the art for articles formed from fluoropolymers, which articles have good properties that are acceptable for storing moisture sensitive products. Such properties include providing chemical resistance, improved product shelf life and eliminating the breakage concerns of glass containers.

As just one example, there is a desire from many end-users to switch from glass containers to something more durable. However, in the pharmaceutical space, currently available containers do not have the needed chemical resistance combined with the necessary ability to be sterilized without degradation, loss in clarity, and loss in structural integrity. Those of skill in the art appreciate that commonly used plastics, such as HDPE, PET, PVC, PP, PS (polystyrene) and polylactides, have varying physical properties which are not ideal for use in the aforementioned molded articles.

As just one example, although PET can be used in an article, it undesirably reacts with many solvents and pharmaceuticals and thereby cannot be used in many applications. Similarly, some other polymers can also be used but tend to yellow or otherwise degrade when sterilized. Therefore, these polymers also typically cannot be used in many applications. Still further, and as introduced above, there are known issues with adhesion of various polymers to one another. For all of these reasons, there are many problems to be solved in this area.

Accordingly, there remains an opportunity for improvement and solution to such problems. Other desirable features and characteristics of the present disclosure will become apparent from the subsequent detailed description of the disclosure and the appended claims, taken in conjunction with the accompanying drawings and this background of the disclosure.

### BRIEF SUMMARY

This disclosure provides a multi-layer article for storing a product, such as a pharmaceutical product. The article includes a first layer that includes a polychlorotrifluoroethylene and/or copolymer of polychlorotrifluoroethylene and vinylidene fluoride. The article also includes a tie layer disposed on the first layer. The tie layer consists essentially of an ethylene acrylate resin. The article further includes a second layer that is disposed on the tie layer. The second layer includes a polyester, polycarbonate, polypropylene, polyethylene, or combinations thereof. The article further optionally includes two optional layers each independently including a polychlorotrifluoroethylene and/or copolymer of polychlorotrifluoroethylene and vinylidene fluoride, or an ethylene acrylate resin, or a polyester, polycarbonate, polypropylene, polyethylene, or combinations thereof. The article has a clarity of at least about 90% as determined using ASTM D1003-13.

This disclosure also provides a multi-layer article for storing an animal health pharmaceutical. This article includes the first layer that is disposed to contact the animal health pharmaceutical and that is a polychlorotrifluoroethylene. The article also includes the tie layer disposed on and in direct contact with the first layer and that is an ester modified ethylene acrylate resin. The article further includes the second layer disposed on and in direct contact with that tie layer and that is polyethylene terephthalate. In this embodiment, the innermost layer has a thickness of from about 0.013 to 0.15 mm (0.5 to about 6 mils), the tie layer has a thickness of from about 0.013 to 0.076 mm (0.5 to about 3 mils), and the outermost layer has a thickness of from about 0.13 to 0.64 mm (5 to about 25 mils). Moreover, in this embodiment, the article has a clarity of at least about 90% as determined using ASTM D1003-13.

This disclosure further provides a method of gamma sterilizing the multi-layer article. The method includes the step of providing the article and gamma sterilizing the multi-layer article.

In various embodiments, this disclosure provides a commercial quality rigid/flexible container that can be co-extruded with PCTFE to keep cost competitive while maintaining properties like chemical resistance, gamma sterilizable, clarity, moisture barrier, improved oxygen barrier when compared to most NBA materials and lighter weight and fragility as compared to a glass equivalent.

### BRIEF DESCRIPTION OF THE DRAWINGS

The present disclosure will hereinafter be described in conjunction with the following drawing figures, wherein like numerals denote like elements, and
FIG. 1 is a plan-view of one embodiment of the multi-layer article of this disclosure;
FIG. 2 is a plan-view of another embodiment of the multi-layer article of this disclosure; and
FIG. 3 is a plan-view of another embodiment of the multi-layer article of this disclosure, wherein the article is a bottle.

### DETAILED DESCRIPTION

The following detailed description is merely exemplary in nature and is not intended to limit the multi-layer article. Furthermore, there is no intention to be bound by any theory presented in the preceding background or the following detailed description.

Embodiments of the present disclosure are generally directed to three layer articles and methods for fabricating the same. For the sake of brevity, conventional techniques related to formation of polymers and general multi-layer articles may not be described in detail herein. Moreover, the various tasks and process steps described herein may be incorporated into a more comprehensive procedure or process having additional steps or functionality not described in detail herein. In particular, various steps in the manufacture of polymers and general multi-layer articles are well-known and so, in the interest of brevity, many conventional steps will only be mentioned briefly herein or will be omitted entirely without providing the well-known process details.

### Article

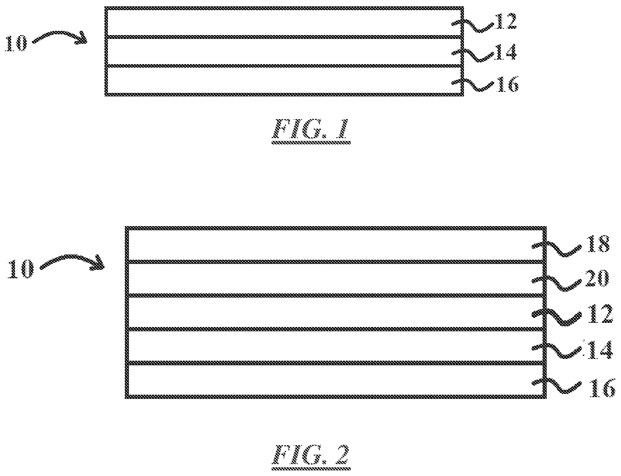

This disclosure provides a multi-layer article (10), as is shown in FIGS. 1-3. The article (10) may be further defined as a film, container, box, carton, bag, envelope, drum, can, bottle, or the like. The article (10) may be further defined as having a shape known in the art as packer, jar, oblong, round, oval, cylinder, sprayer, bear, canister, tottle, etc.

The dimensions of the article (10) are not particularly limited and may be chosen by one of skill in the art. In various embodiments, the volume of the article (10) is from about 15ml to about 125ml (0.5oz to about 4oz) , about (120ml to about 250ml) (about 4oz to about 8oz) (), about 250ml to about 355ml (about 8oz to about 12oz) , about 350ml to about 500ml (about 12oz to about 16oz) , about 490ml to about 600ml (about 16oz to about 20oz) , about 600ml to about 710ml (about 20oz to about 24oz) , about 700ml to about 1000ml (about 24oz to about 32oz) , about 950ml to about 2000ml (about 32oz to about 64oz) , about 1950ml to about 3000ml (about 64oz to about 100oz) , about 2900ml to about 4000ml (about 100oz to about 136oz) , or greater than about 4000ml. In various non-limiting embodiments, all values and ranges of values including and between those set forth above are hereby expressly contemplated for use herein.

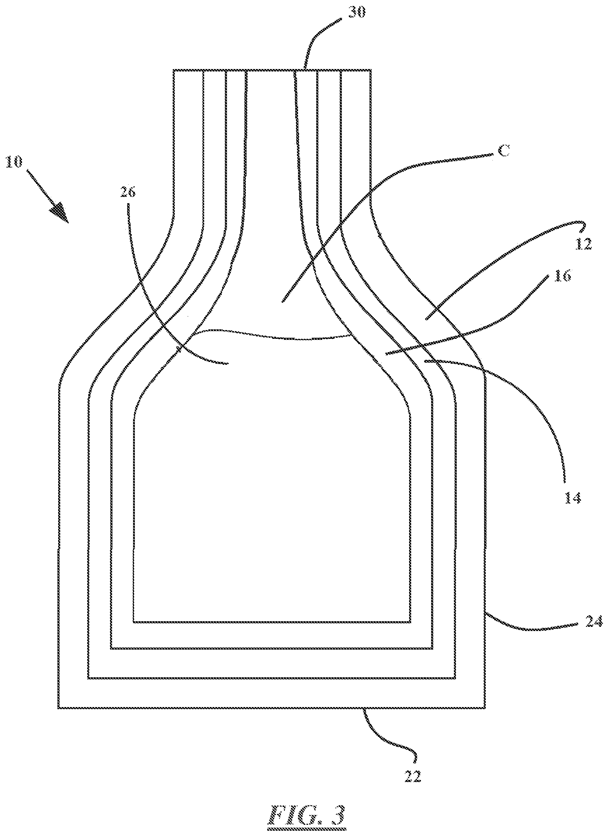

In one embodiment, the article (10) is further defined as a bottle, as shown in FIG. 3. For example, the article (10) may have a wide mouth large enough for pills and tablets and have a mouth size of from about 28mm to about 120mm. Narrow neck articles have neck diameters from about 15mm to about 28mm and are typically used for liquids. In various non-limiting embodiments, all values and ranges of values including and between those set forth above are hereby expressly contemplated for use herein.

The article (10) is designed to store a product (26). The product (26) may be a liquid, solid, or gas. In various embodiments, the product (26) is a liquid, such as a liquid pharmaceutical, such as an animal health pharmaceutical. Alternatively, the product (26) may be a solvent, e.g. a polar or non-polar solvent. The product (26) may be water.

Alternatively, the product (26) may be a solid, such as a pharmaceutical tablet, caplet, etc. In still further embodiments, the product (26) may be a medical device, such as an implant, re-constructive prosthesis, subcutaneous drug delivery device such as a medical implantable pill, a drug-eluting stent, and the like. In yet other embodiments, the product (26) is further defined as a trauma device, surgical tool, plate, surgical fixation device, surgical accessory, or the like. The surgical fixation device may include, but is not limited to, screws, pins, and fasteners. In still another embodiment, the product (26) is an animal health pharmaceutical, e.g. a liquid pharmaceutical. In further embodiments, the product (26) is caplets or capsules, liquids or lotions, powders or granules, condiments or sauces, beverages, or the like.

The article (10) is multi-layered. For example, the article (10) typically includes a total of three or five layers, as described below. In one embodiment, the article (10) consists of three layers (12, 14, 16), e.g. as shown in FIG. 1 and 3. In another embodiments, the article (10) consists of five layers (12, 14, 16, 18, 20), e.g. as shown in FIG. 2. It is also contemplated that the article (10) may consist of four layers (not shown in the Figures).

The article is typically a container that is defined by walls. As shown in FIG. 3, the layers (12, 14, 16), and optionally layers (18, 20), can form a base (22), side walls (24), and/or end walls (not shown). The layers (12, 14, 16), and optionally layers (18, 20), typically define a cavity (C). The cavity (C) is typically entirely enclosed (i.e., defined on all sides) by the layers (12, 14, 16). The layers (12, 14, 16), and optionally layers (18, 20), may also form a top (30) to entirely enclose the cavity (C). Alternatively, the top (30) may be formed from a different material altogether. The cavity (C) may be defined as any shape including, but not limited to, cylindrical shapes, spherical shapes, conical shapes, rectangular shapes, cubic shapes, and the like. In one embodiment, the cavity (C) is defined as a shape that is the same as, substantially similar to, or complementary to, the shape of the product (26). In another embodiment, the cavity (C) is defined as a pocket. Typically the cavity (C) receives the product (26).

### First Layer

The article (10) includes the first layer (16). The first layer (16) may be described as an innermost layer, an outermost layer, or an interior or exterior layer, of the article (10). In one embodiment, the first layer (16) is an innermost layer that is disposed to contact the product (26). The terminology "innermost" describes that this first layer (16) faces the product (26) that is held by the article (10). For example, in such an embodiment, there is no layer disposed on top of the first layer (16) to contact the product (26) that is held by the article (10). The first layer (16) may be alternatively described as the inside-most, inside, or inner, layer. The first layer (16) be alternatively described as an interior layer or layer which contacts the product (26) or which is designed to contact the product (26). In one embodiment, the first layer (16) is exposed to an interior environment of the article (10), e.g. the cavity (C) shown in FIG. 3. The first layer (16) as innermost layer may be disposed opposite the second layer (12) as outermost layer.

Alternatively, the first layer (16) may be an outermost layer such that it is disposed opposite the product (26). The terminology "outermost" describes that this first layer (16) faces the environment and is disposed opposite the second layer (12), which itself could then be an innermost layer. For example, in such an embodiment, there is no layer disposed on top of the second layer (12) to face the environment. The first and/or second layers (16, 12) may be alternatively described as the outside-most, outside, or outer, layer, e.g. a layer that is handled and touched by the consumer. The second layer (12) be alternatively described as an exterior layer or layer which is exposed to the environment.

The first layer (16) includes a polychlorotrifluoroethylene (PCTFE) and/or copolymer of polychlorotrifluoroethylene and vinylidene fluoride. The PCTFE may be neutralized or non-neutralized. For example, the neutralized form of the PCTFE may be as described in US 9,862,811. In one embodiment, the first layer (16) is neutralized PCTFE. In another embodiment, the first layer (16) is non-neutralized PCTFE. In still another embodiment, the first layer (16) is a mixture of neutralized and non-neutralized PCTFE. In still other embodiments, the first layer (16) may include the copolymer of the PCTFE and vinylidene fluoride (VF). Typically, the copolymer may include up to about 5, 4, 3, 2, 1, 0.5, or 0.1, weight percent of the VF. Moreover, the copolymer may include any value or range of values of weight percents of the VF including and between those values described above, e.g. 1-5 wt% of the VF in the copolymer. The first layer (16) is typically about 50 to about 100, about 55 to about 95, about 60 to about 90, about 65 to about 85, about 70 to about 80, about 75 to about 80, or about 90, 91, 92, 93, 94, 95, 96, 97, 98, 99, or even higher, weight percent of the polychlorotrifluoroethylene (PCTFE) (neutralized and/or non-neutralized) and/or copolymer of polychlorotrifluoroethylene and vinylidene fluoride. In various non-limiting embodiments, all values and ranges of values including and between those set forth above are hereby expressly contemplated for use herein.

The first layer (16) may be, include, consist essentially of, or consist of, the polychlorotrifluoroethylene (PCTFE) (neutralized and/or non-neutralized) and/or copolymer of polychlorotrifluoroethylene and vinylidene fluoride. The terminology "consists essentially of" describes an embodiment wherein the first layer (16) is free of other polymers not described above or includes less than 5, 4, 3, 2, 1, 0.5, or 0.1, weight percent of those other polymers. The first layer (16) may be free of the copolymer and/or one of the two types of PCTFE. In various non-limiting embodiments, all values and ranges of values including and between those set forth above are hereby expressly contemplated for use herein.

### Tie Layer

The article (10) also includes the tie layer (14) disposed on the first layer (16). The tie layer (14) may be disposed on and in direct contact with the first layer (16), e.g. as shown in FIG. 1. In other words, in such an embodiment, there is no other layer disposed between the tie layer (14) and the first layer (16). In this embodiment, the tie layer (14) touches the first layer (16). Alternatively, the tie layer (14) may be disposed on and spaced apart from the first layer (16), e.g. with one or more layers disposed in-between such that the tie layer (14) does not directly touch the first layer (16).

The tie layer (14) consists essentially of an ethylene acrylate resin. The ethylene acrylate resin may be alternatively described as a modified ethylene acrylate resin, which may be any known in the art. For example, the modified ethylene acrylate resin may be modified with esters, anhydrides, alkanes, alkenes, ketones, aldehydes, amines, etc. In one embodiment, the tie layer is an ester modified ethylene acrylate resin. A suitable non-limiting example is BYNEL^{®} Series 2200 resins which are ester modified ethylene acrylate resins. They contain a temperature stable ester which makes them functional in high temperature coextrusions.

The tie layer (14) is typically about 50 to about 100, about 55 to about 95, about 60 to about 90, about 65 to about 85, about 70 to about 80, about 75 to about 80, or about 90, 91, 92, 93, 94, 95, 96, 97, 98, 99, or even higher, weight percent of the (modified) ethylene acrylate. The tie layer (14) may be, include, consist essentially of, or consist of, the (modified) ethylene acrylate. The terminology "consists essentially of" describes an embodiment wherein the tie layer (14) is free of other polymers not described above or includes less than 5, 4, 3, 2, 1, 0.5, or 0.1, weight percent of those other polymers. In various non-limiting embodiments, all values and ranges of values including and between those set forth above are hereby expressly contemplated for use herein.

### Second Layer

The article (10) further includes the second layer (12). The second layer (12) may be an innermost or outermost layer, e.g. as described above. For example, if the second layer (12) is an outermost layer, the terminology "outermost" describes that this second layer (12) faces the environment and is disposed opposite the first layer (16). In such an embodiment, there is no layer disposed on top of the second layer (12) to face the environment. The second layer (12) may be alternatively described as the outside-most, outside, or outer, layer, e.g. a layer that is handled and touched by the consumer. The second layer (12) be alternatively described as an exterior layer or layer which is exposed to the environment.

The second layer (12) is disposed on the tie layer (14). In one embodiment, the second layer (12) is disposed on and in direct contact with the tie layer (14), e.g. as shown in FIG. 1. In other words, in such an embodiment, there is no other layer disposed between the tie layer (14) and the second layer (12). In this embodiment, the tie layer (14) touches the second layer (12). For example, the second layer (12) and the first layer (16) sandwich the tie layer (14) therebetween thereby forming a multi-layer article (10), e.g. as shown in FIGS. 1 and 3. Alternatively, the second layer (12) may be disposed on and spaced apart from the tie layer (14), e.g. with one or more layers disposed in-between such that the tie layer (14) does not directly touch the second layer (12).

The second layer (12) includes a polyester, polycarbonate, polypropylene, polyethylene, or combinations thereof. The polyester is not particularly limited and may be any known in the art. In various embodiments, the polyester is chosen from polyglycolide or polyglycolic acid (PGA), polylactic acid (PLA), polycaprolactone (PCL), polyhydroxyalkanoate (PHA), polyhydroxybutyrate (PHB), polyethylene adipate (PEA), polybutylene succinate (PBS), poly(3-hydroxybutyrate-co-3-hydroxyvalerate) (PHBV), polyethylene terephthalate (PET), polybutylene terephthalate (PBT), polytrimethylene terephthalate (PTT), polyethylene naphthalate (PEN), or combinations thereof. In one embodiment, the polyester is PET. In another embodiment, the second layer (12) is the polycarbonate. In a further embodiment, the second layer (12) is polypropylene. In yet another embodiments, the second layer (12) is polyethylene, e.g. Ultra-high-molecular-weight polyethylene (UHMWPE); Ultra-low-molecular-weight polyethylene (ULMWPE or PE-WAX); High-molecular-weight polyethylene (HMWPE); High-density polyethylene (HDPE); High-density cross-linked polyethylene (HDXLPE); Cross-linked polyethylene (PEX or XLPE); Medium-density polyethylene (MDPE); Linear low-density polyethylene (LLDPE); Low-density polyethylene (LDPE); Very-low-density polyethylene (VLDPE); or Chlorinated polyethylene (CPE); and/or combinations thereof.

The polyester for the second layer (12) may be chosen based on intrinsic viscosity. The polyester may have an intrinsic of from about 1 to about 1.05, about 1 to about 1.1, about 1 to about 1.15, about 1 to about 1.2, about 1.05 to about 1.2, about 1.05 to about 1.15, about 1.05 to about 1.1, about 1.1 to about 1.2, about 1.1 to about 1.15, about 1.15 to about 1.2, etc., as determined using DAK-QAR-SOP-0012. In various embodiments, these values can vary by ±0.01, 0.02, 0.03, 0.04, 0.05, 0.06, 0.07, 0.08, 0.09, or 0.1. In various non-limiting embodiments, all values and ranges of values including and between those set forth above are hereby expressly contemplated for use herein.

The second layer (12) is typically about 50 to about 100, about 55 to about 95, about 60 to about 90, about 65 to about 85, about 70 to about 80, about 75 to about 80, or about 90, 91, 92, 93, 94, 95, 96, 97, 98, 99, or even higher, weight percent of the polyester, polycarbonate, polypropylene, polyethylene, or combinations thereof. The second layer (12) may be, include, consist essentially of, or consist of, the polyester, polycarbonate, polypropylene, polyethylene, or combinations thereof. The terminology "consists essentially of" describes an embodiment wherein the second layer (12) is free of other polymers not described above or includes less than 5, 4, 3, 2, 1, 0.5, or 0.1, weight percent of those other polymers. In various non-limiting embodiments, all values and ranges of values including and between those set forth above are hereby expressly contemplated for use herein.

### Optional Layers

The article (10) also optionally includes two optional layers (18, 20) each independently including a polychlorotrifluoroethylene and/or copolymer of polychlorotrifluoroethylene and vinylidene fluoride, or an ethylene acrylate resin, or a polyester, polycarbonate, polypropylene, polyethylene, or combinations thereof. For example, one or both of the optional layers may or may not be included in the article (10). One or both of the optional layers may be the same as, or similar to, any one or more of the aforementioned layers (12, 14, 16). Alternatively, they may be different from any one or more of the aforementioned layers (12, 14, 16). In various embodiments, both a first optional layer (18) and a second optional layer (20) are disposed on the first layer (16), e.g. as shown in FIG. 2.

Although each of the aforementioned layers (12, 14, 16, 18, 20) can have a different thickness, first layer typically has a thickness of from about 0.5 to about 6 mils, while the tie layer typically has a thickness of from about 0.5 to about 3 mils, and the second layer typically has a thickness of from about 5 to about 25 mils. The first and second optional layers (18, 20), if included, can have the same or different thickness as any one of the layers (12, 14, 16). For example, the first and second optional layers (18,20) may have the same or different thicknesses than each other, e.g. from about 0.013 to about 0.64 mm (about 0.5 to about 25 mils). In various embodiments, any one or more of the aforementioned layers (12, 14, 16, 18, 20) can have a thickness of from about 0.013 to about 0.14 mm (about 0.5 to about 5.5), about 0.25 mm to about 0.13 mm (about 1 to about 5), about 0.38 to about 0.11 mm (about 1.5 to about 4.5), about 0.051 to about 0.10 mm (about 2 to about 4), about 0.064 to about 0.089 mm (about 2.5 to about 3.5), about 0.064 to about 0.10 mm (about 2.5 to about 4), about 0.025 to about 0.064 mm (about 1 to about 2.5), about 0.038 to about 0.051 mm (about 1.5 to about 2), about 0.13 to about 0.51 mm (about 5 to about 20), about 0.25 to about 0.38 mm (about 10 to about 15), about 0.38 to about 0.51 mm (about 15 to about 20), or about 0.025, 0.051, 0.076, 0.10, 0.13, 0.15, 0.18, 0.20, 0.23, 0.25, 0.28, 0.30, 0.33, 0.36, 0.38, 0.41, 0.43, 0.46, 0.48, 0.51, 0.53, 0.56, 0.58, 0.61, 0.64 mm (about 1, 2, 3, 4, 5, 6, 7, 8, 9, 10, 11, 12, 13, 14, 15, 16, 17, 18, 19, 20, 21, 22, 23, 24, or 25, mils). In various non-limiting embodiments, all values and ranges of values including and between those set forth above are hereby expressly contemplated for use herein.

In addition to the above, the PCTFE of the article (10) has a USP Class VI designation. As is known in the art, the United States Pharmacopeia (USP) is a non-government organization that endorses public health by establishing standards to safeguard the quality of medicines and other health care technologies. The USP Standards are published in the US Pharmacopeia and the National Formulary (USP NF). USP Class VI products go through a series of biological tests. The USP Class VI compounds must be made from ingredients with clear histories of biocompatibility that meet tight requirements for leachates. These tests include an Acute Systemic Toxicity Test, an Intracutaneous Test, an Implantation Test, a Standard Temperatures and Times test, etc. There are three in vivo tests included in this classification including the Systemic Injection Test and the Intracutaneous Test. A plastic resin material that has passed Class VI certification is expected to be more likely to produce favorable biocompatibility results. For a product to pass USP Class VI standards, it must exhibit a very low level of toxicity by passing all of the test requirements. Compliance to USP Class VI is often requested by end users. Testing for compliance involves an assessment of the effects of the material, and extractables, on tissue.

In addition, the article (10) has a clarity of at least about 90, 91, 92, 93, 94, 95, 96, 97, 98, 99, or about 100, percent, as determined using ASTM D1003-13. In other embodiments, the article (10) has a transmission of at least about 90, 91, 92, 93, 94, 95, 96, 97, 98, 99, or about 100, percent, as determined using ASTM D1003-13. In still other embodiments, the article (10) has a haze of less than 2, 1.9, 1.8, 1.7, 1.6, 1.5, 1.4, 1.3, 1.2, 1.1, 1, 0.9, 0.8, 0.7, 0.6, 0.5, 0.4, 0.3, 0.2, or 0.1, percent, as determined using ASTM D1003-13. In various non-limiting embodiments, all values and ranges of values including and between those set forth above are hereby expressly contemplated for use herein.

The moisture vapor transmission rate (MVTR) of the article (10) may be determined via the procedure set forth in ASTM F1249. In one embodiment, the article (10) has a typical MVTR of from about 15.5 or less g/m²/day (1.0 or less g/100 in²/day) of the overall structure at 37.8°C. and 100% relative humidity (RH), more typically from 0.0077 to about 10.7 g/m²/day (0.0005 to about 0.7 g/100 in²/day) of the overall structure, and most typically from 0.015 to about 0.93 g/m²/day (0.001 to about 0.06 g/100 in²/day) of the overall structure, as determined by water vapor transmission rate measuring equipment available from, for example, Mocon. In various non-limiting embodiments, all values and ranges of values including and between those set forth above are hereby expressly contemplated for use herein.

The oxygen transmission rate (OTR) of the article (10) may be determined via the procedure of ASTM D-3985 using an OX-TRAN 2/20 instrument manufactured by Mocon, operated at 25°C, 0% RH. In one embodiment, the article (10) has an OTR of from about 775 or less g/m²/day (50 or less cc/100 in²/day) , more typically from about 0.015 to about 310 g/m²/day (0.001 to about 20 cc/100 in²/day) , and most typically from about 0.015 to about 150 cc/m²/day (0.001 to about 10 cc/100 in²/day) . In various non-limiting embodiments, all values and ranges of values including and between those set forth above are hereby expressly contemplated for use herein.

Each of the aforementioned layers (12, 14, 16, 18, 20) may include, or be free of, one or more conventional additives whose uses are well known to those skilled in the art. The use of such additives may be desirable in enhancing the processing of the layers (12, 14, 16, 18, 20). Examples of such include oxidative and thermal stabilizers, lubricants, release agents, flame-retarding agents, oxidation inhibitors, oxidation scavengers, dyes, pigments and other coloring agents, ultraviolet light absorbers and stabilizers, organic or inorganic fillers including particulate and fibrous fillers, reinforcing agents, nucleators, plasticizers, as well as other conventional additives known to the art. Such may be used in amounts, for example, of up to about 30% by weight of the overall layer composition. Representative ultraviolet light stabilizers include various substituted resorcinols, salicylates, benzotriazoles, benzophenones, and the like. Suitable lubricants and release agents include wax, stearic acid, stearyl alcohol, and stearamides. Exemplary flame-retardants include organic halogenated compounds, including decabromodiphenyl ether and the like as well as inorganic compounds. Suitable coloring agents including dyes and pigments include cadmium sulfide, cadmium selenide, titanium dioxide, phthalocyanines, ultramarine blue, nigrosine, carbon black and the like. Representative oxidative and thermal stabilizers include the Period Table of Element's Group I metal halides, such as sodium halides, potassium halides, lithium halides; as well as cuprous halides; and further, chlorides, bromides, iodides. Also acceptable are hindered phenols, hydroquinones, aromatic amines as well as substituted members of those above mentioned groups and combinations thereof. Exemplary plasticizers include lactams such as caprolactam and lauryl lactam, sulfonamides such as o,p-toluenesulfonamide and N-ethyl, N-butyl benylenesulfonamide, and combinations of any of the above, as well as other plasticizers known to the art.

### Additional Embodiments

In one embodiment, the article (10) is free of the two optional layers and is a three-layered structure wherein the tie layer is disposed in direct contact with the first layer and the second layer is disposed in direct contact with the tie layer. In another embodiment, the first layer (16) is an innermost layer disposed to contact the product (26) and the second layer (12) is an outermost layer. In a further embodiment, the second layer (12) is an innermost layer disposed to contact the product (26) and the first layer (16) is an outermost layer.

Alternatively, the article (10) includes the two optional layers (18, 20) and is a five-layered structure wherein both the first optional layer (18) and a second optional layer (20) are disposed on the first layer (16), wherein the first optional layer (16) includes a polyester, polycarbonate, polypropylene, polyethylene, or combinations thereof and sandwiches the second optional layer (20) between the first optional layer (18) and the first layer (16), and wherein the second optional layer (20) comprises an ethylene acrylate resin.

In various related embodiments, the first layer (16) is polychlorotrifluoroethylene. The polychlorotrifluoroethylene may be neutralized or non-neutralized. Alternatively, the first layer (16) can include or be the copolymer of polychlorotrifluoroethylene and up to 5 wt% of vinylidene fluoride. In still other related embodiments, the ethylene acrylate resin is an ester modified ethylene acrylate resin. Moreover, the second layer (12) may be a polyester such as polyethylene terephthalate (PET). Moreover, in still other related embodiments, the first layer (16) has a thickness of from about 0.013 to 0.15 mm (0.5 to about 6 mils), the tie layer (14) has a thickness of from about 0.013 to 0.076 mm (0.5 to about 3 mils), and the second layer (12) has a thickness of from about 0.13 to 0.64 mm (5 to about 25 mils). Similarly, the two optional layers (18,20) can each independently have a thickness of from about 0.013 to 0.64 mm (0.5 to about 25 mils). In various non-limiting embodiments, all values and ranges of values including and between those set forth above are hereby expressly contemplated for use herein.

In one embodiment, the article (10) is a three-layer article for storing an animal health pharmaceutical. In one embodiment, the article (10) consists of an innermost layer (16) that is disposed to contact the animal health pharmaceutical (26) and that is a polychlorotrifluoroethylene; a tie layer (14) disposed on and in direct contact with the first layer (16) and that is an ester modified ethylene acrylate resin; and an outermost layer (12) disposed on and in direct contact with said tie layer (14) and that is polyethylene terephthalate. In this embodiment, said innermost layer (16) has a thickness of from about 0.013 to 0.15 mm (0.5 to about 6 mils), said tie layer (14) has a thickness of from about 0.013 to 0.076 mm (0.5 to about 3 mils), and said outermost layer (12) has a thickness of from about 0.13 to 0.64 mm (5 to about 25 mils). Moreover, in this embodiment, the article (10) has a clarity of at least about 90% as determined using ASTM D1003-13. For example, this article (10) can be further defined as a bottle, e.g. as shown in FIG. 3. In various non-limiting embodiments, all values and ranges of values including and between those set forth above are hereby expressly contemplated for use herein.

In still other embodiments, the article (10) has a three-layer structure as follows:
Innermost layer of PCTFE/tie layer/outermost layer of PC, PET, HDPE, PP; or
Outermost layer of PCTFE/tie layer/innermost layer of PC, PET, HDPE, PP.

In other embodiments, the article (10) has a five-layer structure as follows:
Innermost layer of PC, PET, HDPE, PP/tie layer/PCTFE/tie layer/outermost layer of PC, PET, HDPE, PP; or
Outermost layer of PC, PET, HDPE, PP/tie layer/PCTFE/tie layer/innermost layer of PC, PET, HDPE, PP.

In even further embodiments, the article (10) may have the following layer distribution wherein all values are in weight percent based on the weight of the article (10) as a whole:

### Three-layer structure having PCTFE as an innermost layer:

Innermost layer of PCTFE: about 5 to about 35, about 10 to about 30, about 15 to about 25, or about 15 to about 20, weight percent based on a total weight of the article (10);
Tie layer: about 5 to about 15, about 10 to about 15 or about 5 to about 10, weight percent based on a total weight of the article (10);
Outermost layer: about 45 to about 75, about 50 to about 70, about 55 to about 65, or about 55 to about 60, weight percent based on a total weight of the article (10).

### Three-layer structure having PCTFE as an outermost layer:

Innermost layer of PC, PET, HDPE, PP: about 45 to about 75, about 50 to about 70, about 55 to about 65, or about 55 to about 60, weight percent based on a total weight of the article (10);
Tie layer: about 5 to about 15, about 10 to about 15 or about 5 to about 10, weight percent based on a total weight of the article (10);
Outermost layer of PCTFE: about 5 to about 35, about 10 to about 30, about 15 to about 25, or about 15 to about 20, weight percent based on a total weight of the article (10).

### Five-layer structure:

Innermost layer of PC, PET, HDPE, PP: about 25 to about 75, about 30 to about 70, about 35 to about 65, about 40 to about 60, about 45 to about 55, or about 45 to about 50, weight percent based on a total weight of the article (10);
Tie layer: about 5 to about 15, about 10 to about 15 or about 5 to about 10, weight percent based on a total weight of the article (10);
PCTFE layer: about 5 to about 35, about 10 to about 30, about 15 to about 25, or about 15 to about 20, weight percent based on a total weight of the article (10);
Tie layer: about 5 to about 15, about 10 to about 15 or about 5 to about 10, weight percent based on a total weight of the article (10); and
Outermost layer of PC, PET, HDPE, PP: about 25 to about 75, about 30 to about 70, about 35 to about 65, about 40 to about 60, about 45 to about 55, or about 45 to about 50, weight percent based on a total weight of the article (10).

In various non-limiting embodiments, all values and ranges of values including and between those set forth above are hereby expressly contemplated for use herein.

### Methods of Forming the Article

The article (10) may be produced by conventional methods known in the art, including well known injection molding, co-injection blow molding, co-injection stretch-blow molding or co-extrusion blow molding techniques. For example, the article (10) may be formed using a method of supplying the components/polymer for one or more of the layers and then forming the article (10) around a suitable mold and heating in a method well known in the art.

The most typical method for producing the article (10) is co-extrusion blow molding. Co-extrusion blow molding is a process in which individual extruders feed the individual components into a co-extrusion head, where a multi-layer parison is formed and extruded. The parison, still in its melt form, is then sandwiched between two mold halves. Once the mold halves have fully closed on the parison, air is introduced into the center of the parison which in turn expands the parison into the configuration of the mold cavity. The material then solidifies due to lower temperatures introduced from the surface of the chilled mold halves. The mold halves are then opened up and the newly formed container is ejected from the mold.

Alternately, the article (10) may be formed by first combining the individual layers described herein to form a multilayered film, followed by subsequently shaping the multilayered film into a desired shaped article (10). In this embodiment, multilayered films may be produced by conventional methods useful in producing multilayer films, including coextrusion and lamination techniques. In a conventional coextrusion process, the polymeric material for the individual layers are fed into infeed hoppers of a like number of extruders, each extruder handling the material for one or more of the layers. The melted and plasticized streams from the individual extruders are directly fed to a multi-manifold die and then juxtaposed and combined into a layered structure or combined into a layered structure in a combining block and then fed into a single manifold or multi-manifold co-extrusion die. The layers emerge from the die as a single multiple layer film of polymeric material. After exiting the die, the film is cast onto a first controlled temperature casting roll, passes around the first roll, and then onto a second controlled temperature roll. The controlled temperature rolls largely control the rate of cooling of the film after it exits the die. Additional rolls may be employed. In another method, the film forming apparatus may be one which is referred to in the art as a blown film apparatus and includes a multi-manifold circular die head for bubble blown film through which the plasticized film composition is forced and formed into a film bubble which may ultimately be collapsed and formed into a film. One advantage of coextruded films is the formation of a multilayer film in a one process step by combining molten layers of each of the film layers, as well as any other optional film layers, into a unitary film structure.

Alternately, individual layers may first be formed as separate layers and then laminated together under heat and pressure with or without intermediate adhesive layers. Lamination techniques are well known in the art. Typically, laminating is done by positioning the individual layers on one another under conditions of sufficient heat and pressure to cause the layers to combine into a unitary film. Typically the layers are positioned on one another, and the combination is passed through the nip of a pair of heated laminating rollers by techniques well known in the art. Lamination heating may be done at temperatures ranging from about 120°C to about 175°C, typically from about 150°C to about 175°C, at pressures of from about 0.034 MPa (5 psig) to about 0.69 MPa (100 psig) , for from about 5 seconds to about 5 minutes, typically from about 30 seconds to about 1 minute. In various non-limiting embodiments, all values and ranges of values including and between those set forth above are hereby expressly contemplated for use herein.

In one embodiment, the article (10) is formed by co-extrusion blow molding. For example, a film may be uniaxially or biaxially oriented prior to molding. The layers may be drawn to a draw ratio of from 1.5:1 to 5:1 uniaxially in at least one direction, i.e. its longitudinal direction, its transverse direction or biaxially in each of its longitudinal and transverse directions. The term draw ratio is an indication of the increase in the dimension in the direction of draw. For example, the article (10) may be uniaxially oriented from about 1.3 to about 10 times in either its longitudinal or transverse directions, or the multilayered structure may be biaxially oriented from about 1.5 to about 5 times each of its longitudinal and transverse directions. The article (10) may also be drawn to a lesser or greater degree in either or both of said longitudinal and transverse directions. The layers may be simultaneously biaxially oriented, for example orienting a film in both the machine and transverse directions at the same. This results in dramatic improvements in clarity, strength and toughness properties, as well as an improved moisture vapor transmission rate. In various non-limiting embodiments, all values and ranges of values including and between those set forth above are hereby expressly contemplated for use herein.

### A Method of Gamma Sterilizing the Article

This disclosure also provides a method of gamma sterilizing the article (10). The method includes the step of providing the article (10), which may be any described above, and gamma sterilizing the article (10). The step of gamma sterilizing may be utilized using any conditions chosen by one of skill in the art.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration in any way. Rather, the foregoing detailed description will provide those skilled in the art with a convenient road map for implementing an exemplary embodiment. It being understood that various changes may be made in the function and arrangement of elements described in an exemplary embodiment without departing from the scope as set forth in the appended claims.

## Claims

1. A multi-layer article for storing a product, said article comprising:
A. a first layer comprising a polychlorotrifluoroethylene and/or copolymer of polychlorotrifluoroethylene and vinylidene fluoride;
B. a tie layer disposed on said first layer and consisting essentially of an ethylene acrylate resin; and
C. a second layer disposed on said tie layer and comprising a polyester, polycarbonate, polypropylene, polyethylene, or combinations thereof, and
D.
wherein said article is a three-layered structure wherein said tie layer is disposed in direct contact with said first layer and said second layer is disposed in direct contact with said tie layer; and
wherein said article has a clarity of at least 90% as determined using ASTM D1003-13.

2. The article of claim 1 wherein the first layer is an innermost layer disposed to contact the product and the second layer is an outermost layer.

3. The article of claim 1 wherein the second layer is an innermost layer disposed to contact the product and the first layer is an outermost layer.

4. The article of claim 1 that also comprises two optional layers and is a five-layered structure,
wherein both a first optional layer and a second optional layer are disposed on said first layer,
wherein the first optional layer comprises a polyester, polycarbonate, polypropylene, polyethylene, or combinations thereof and sandwiches the second optional layer between the first optional layer and the first layer, and
wherein the second optional layer comprises an ethylene acrylate resin.

5. The article of any one of claims 1-4 wherein said first layer comprises the copolymer of polychlorotrifluoroethylene and up to 5 wt% of vinylidene fluoride and wherein the ethylene acrylate resin is an ester modified ethylene acrylate resin.

6. The article of any one of claims 1-5 wherein said first layer has a thickness of from 0.013 to 0.15 mm (0.5 to 6 mils), said tie layer has a thickness of from 0.013 to 0.076 mm (0.5 to 3 mils), and said second layer has a thickness of from 0.13 to 0.64 mm (5 to 25 mils).

7. The article of claim 1 that is a three-layer article for storing an animal health pharmaceutical, said article consisting of:
A. the first layer that is an innermost layer that is disposed to contact the animal health pharmaceutical and that is the polychlorotrifluoroethylene;
B. the tie layer disposed on and in direct contact with said first layer and that is the ester modified ethylene acrylate resin; and
C. the second layer is outermost layer disposed on and in direct contact with said tie layer and that is a polyester that is polyethylene terephthalate,
wherein said innermost layer has a thickness of from 0.013 to 0.15 mm (0.5 to 6 mils), said tie layer has a thickness of from 0.013 to 0.076 mm (0.5 to 3 mils), and said outermost layer has a thickness of from 0.13 to 0.64 mm (5 to 25 mils), and
wherein said article has a clarity of at least about 90% as determined using ASTM D1003-13.

8. The article of claim 7 that is further defined as a bottle.

9. A method of gamma sterilizing a multi-layer article, said method comprising the steps of:
A. providing the multi-layer article comprising a first layer comprising a polychlorotrifluoroethylene and/or copolymer of polychlorotrifluoroethylene and vinylidene fluoride, a tie layer disposed on said first layer and consisting essentially of an ethylene acrylate resin, a second layer disposed on said tie layer and comprising a polyester, polycarbonate, polypropylene, polyethylene, or combinations thereof, and two optional layers each independently comprising a polychlorotrifluoroethylene and/or copolymer of polychlorotrifluoroethylene and vinylidene fluoride, or an ethylene acrylate resin, or a polyester, polycarbonate, polypropylene, polyethylene, or combinations thereof, wherein the multi-layer article has a clarity of at least 90% as determined using ASTM D1003-13; and
B. gamma sterilizing the multi-layer article.

## Patentansprüche

1. Mehrschichtartikel zum Aufbewahren eines Produkts, der Artikel umfassend:
A. eine erste Schicht, umfassend ein Polychlortrifluorethylen und/oder ein Copolymer von Polychlortrifluorethylen und Vinylidenfluorid;
B. eine Bindeschicht, die auf die erste Schicht aufgebracht ist und im Wesentlichen aus einem Ethylenacrylatharz besteht; und
C. eine zweite Schicht, die auf die Bindeschicht aufgebracht ist und Polyester, Polycarbonat, Polypropylen, Polyethylen oder Kombinationen davon umfasst, und
D.
wobei der Artikel eine dreischichtige Struktur ist, wobei die Bindeschicht in direktem Kontakt mit der ersten Schicht aufgebracht ist und die zweite Schicht in direktem Kontakt mit der Bindeschicht aufgebracht ist; und
wobei der Artikel eine Lichtdurchlässigkeit von mindestens 90 % aufweist, wie unter Verwendung von ASTM D1003-13 bestimmt.

2. Artikel nach Anspruch 1, wobei die erste Schicht eine innerste Schicht ist, die dazu aufgebracht ist, in Kontakt mit dem Produkt zu sein und die zweite Schicht eine äußerste Schicht ist.

3. Artikel nach Anspruch 1, wobei die zweite Schicht eine innerste Schicht ist, die dazu aufgebracht ist, in Kontakt mit dem Produkt zu sein und die erste Schicht eine äußerste Schicht ist.

4. Artikel nach Anspruch 1, der auch zwei optionale Schichten umfasst und eine fünfschichtige Struktur ist,
wobei sowohl eine erste optionale Schicht als auch eine zweite optionale Schicht auf die erste Schicht aufgebracht sind,
wobei die erste optionale Schicht ein Polyester, Polycarbonat, Polypropylen, Polyethylen oder Kombinationen davon umfasst und die zweite optionale Schicht zwischen der ersten optionalen Schicht und der ersten Schicht sandwichartig angeordnet ist, und
wobei die zweite optionale Schicht ein Ethylenacrylatharz umfasst.

5. Artikel nach einem der Ansprüche 1-4, wobei die erste Schicht das Copolymer von Polychlortrifluorethylen und bis zu 5 Gew.-% Vinylidenfluorid umfasst und wobei das Ethylenacrylatharz ein Ester-modifiziertes Ethylenacrylatharz ist.

6. Artikel nach einem der Ansprüche 1-5, wobei die erste Schicht eine Dicke von 0,013 bis 0,15 mm (0,5 bis 6 mils) aufweist, die Bindeschicht eine Dicke von 0,013 bis 0,076 mm (0,5 bis 3 mils) aufweist und die zweite Schicht eine Dicke von 0,13 bis 0,64 mm (5 bis 25 mils) aufweist.

7. Artikel nach Anspruch 1, der ein Dreischichtartikel zum Aufbewahren eines Tiergesundheitsarzneimittels ist, wobei der Artikel aus Folgendem besteht:
A. der ersten Schicht, die eine innerste Schicht ist, die dazu aufgebracht ist, in Kontakt mit dem Tiergesundheitsarzneimittel zu sein und die das Polychlortrifluorethylen ist;
B. der Bindeschicht, die auf und in direktem Kontakt mit der ersten Schicht aufgebracht ist und die das Ester-modifizierte Ethylenacrylatharz ist; und
C. der zweiten Schicht, die die äußerste Schicht ist, die auf und in direktem Kontakt mit der Bindeschicht aufgebracht ist und die ein Polyester ist, das Polyethylenterephthalat ist,
wobei die innerste Schicht eine Dicke von 0,013 bis 0,15 mm (0,5 bis 6 mils) aufweist, die Bindeschicht eine Dicke von 0,013 bis 0,076 mm (0,5 bis 3 mils) aufweist und die äußerste Schicht eine Dicke von 0,13 bis 0,64 mm (5 bis 25 mils) aufweist, und
wobei der Artikel eine Lichtdurchlässigkeit von mindestens etwa 90 % aufweist, wie unter Verwendung von ASTM D1003-13 bestimmt.

8. Artikel nach Anspruch 7, der ferner als Flasche definiert ist.

9. Verfahren zum Gamma-Sterilisieren eines Mehrschichtartikels, wobei das Verfahren die folgenden Schritte umfasst:
A. Bereitstellen des Mehrschichtartikels, umfassend eine erste Schicht, die ein Polychlortrifluorethylen und/oder ein Copolymer von Polychlortrifluorethylen und Vinylidenfluorid umfasst, eine Bindeschicht, die auf die erste Schicht aufgebracht ist und im Wesentlichen aus einem Ethylenacrylatharz besteht, einer zweiten Schicht, die auf die Bindeschicht aufgebracht ist und ein Polyester, Polycarbonat, Polypropylen, Polyethylen oder Kombinationen davon umfasst und zwei optionale Schichten, die jeweils unabhängig ein Polychlortrifluorethylen und/oder ein Copolymer von Polychlortrifluorethylen und Vinylidenfluorid, oder ein Ethylenacrylatharz oder ein Polyester, Polycarbonat, Polypropylen, Polyethylen oder Kombinationen davon umfassen, wobei der Mehrschichtartikel eine Lichtdurchlässigkeit von mindestens 90 % aufweist, wie unter Verwendung von ASTM D1003-13 bestimmt; und
B. Gamma-Sterilisieren des Mehrschichtartikels.

## Revendications

1. Article multicouche servant à stocker un produit, ledit article comprenant :
A. une première couche comprenant un polychlorotrifluoroéthylène et/ou un copolymère de polychlorotrifluoroéthylène et de fluorure de vinylidène ;
B. une couche de liaison disposée sur ladite première couche et consistant essentiellement en une résine d'acrylate d'éthylène ; et
C. une deuxième couche disposée sur ladite couche de liaison et comprenant un polyester, un polycarbonate, un polypropylène, un polyéthylène ou des combinaisons de ceux-ci, et
D.
dans lequel ledit article est une structure à trois couches dans laquelle ladite couche de liaison est disposée en contact direct avec ladite première couche et ladite deuxième couche est disposée en contact direct avec ladite couche de liaison ; et
dans lequel ledit article a une clarté d'au moins 90 % telle que déterminée en utilisant la norme ASTM D1003-13.

2. Article selon la revendication 1, dans lequel la première couche est une couche la plus interne disposée pour entrer en contact avec le produit et la deuxième couche est une couche la plus externe.

3. Article selon la revendication 1, dans lequel la deuxième couche est une couche la plus interne disposée pour entrer en contact avec le produit et la première couche est une couche la plus externe.

4. Article selon la revendication 1 qui comprend également deux couches facultatives et est une structure à cinq couches,
dans lequel une première couche facultative et une deuxième couche facultative sont disposées sur ladite première couche,
dans lequel la première couche facultative comprend un polyester, un polycarbonate, un polypropylène, un polyéthylène ou une combinaison de ceux-ci et prend en sandwich la deuxième couche facultative entre la première couche facultative et la première couche, et
dans lequel la deuxième couche facultative comprend une résine d'acrylate d'éthylène.

5. Article selon l'une quelconque des revendications 1 à 4, dans lequel ladite première couche comprend le copolymère de polychlorotrifluoroéthylène et jusqu'à 5 % en poids de fluorure de vinylidène et dans lequel la résine d'acrylate d'éthylène est une résine d'acrylate d'éthylène modifiée par un ester.

6. Article selon l'une quelconque des revendications 1 à 5, dans lequel ladite première couche a une épaisseur de 0,013 à 0,15 mm (0,5 à 6 mils), ladite couche de liaison a une épaisseur de 0,013 à 0,076 mm (0,5 à 3 mils) et ladite deuxième couche a une épaisseur de 0,13 à 0,64 mm (5 à 25 mils).

7. Article selon la revendication 1, qui est un article à trois couches servant à stocker un produit pharmaceutique de santé animale, ledit article consistant en :
A. la première couche qui est une couche la plus interne qui est disposée pour entrer en contact avec le produit pharmaceutique de santé animale et qui est le polychlorotrifluoroéthylène ;
B. la couche de liaison disposée sur et en contact direct avec ladite première couche et qui est la résine d'acrylate d'éthylène modifiée par un ester ; et
C. la deuxième couche qui est la couche la plus externe disposée sur et en contact direct avec ladite couche de liaison et qui est un polyester qui est du polyéthylène téréphtalate,
dans lequel ladite couche la plus interne a une épaisseur de 0,013 à 0,15 mm (0,5 à 6 mils), ladite couche de liaison a une épaisseur de 0,013 à 0,076 mm (0,5 à 3 mils) et ladite couche la plus externe a une épaisseur de 0,13 à 0,64 mm (5 à 25 mils), et
dans lequel ledit article a une clarté d'au moins 90 % environ telle que déterminée en utilisant la norme ASTM D1003-13.

8. Article selon la revendication 7 qui est en outre défini comme une bouteille.

9. Procédé de stérilisation gamma d'un article multicouche, ledit procédé comprenant les étapes suivantes :
A. fourniture d'un article multicouche comprenant une première couche comprenant un polychlorotrifluoroéthylène et/ou un copolymère de polychlorotrifluoroéthylène et de fluorure de vinylidène, une couche de liaison disposée sur ladite première couche et consistant essentiellement en une résine d'acrylate d'éthylène, une deuxième couche disposée sur ladite couche de liaison et comprenant un polyester, un polycarbonate, un polypropylène, un polyéthylène ou une combinaison de ceux-ci, et deux couches facultatives comprenant chacune indépendamment un polychlorotrifluoroéthylène et/ou un copolymère de polychlorotrifluoroéthylène et de fluorure de vinylidène, ou une résine d'acrylate d'éthylène, ou un polyester, un polycarbonate, un polypropylène, un polyéthylène ou une combinaison de ceux-ci, dans lequel l'article multicouche a une clarté d'au moins 90 % telle que déterminée en utilisant la norme ASTM D1003-13 ; et
B. stérilisation gamma de l'article multicouche.
